# EUROPÄISCHE PATENTANMELDUNG

(11) **EP 2 903 095 A1**
(43) Veröffentlichungstag der Anmeldung: **05.08.2015**
(21) Anmeldenummer: 15150319.0
(22) Anmeldetag: 07.01.2015
(51) Int. Cl.: H01R 13/187, H01R 13/52, A61N 1/375, H01R 24/58

(54) **Kontaktelement und Verfahren zur Herstellung eines Kontaktelements**

(30) Priorität: 29.01.2014 US 201461932819 P
(71) Anmelder: BIOTRONIK SE & Co. KG, 12359 Berlin (DE)
(72) Erfinder: Starke, Marcel, 15732 Eichwalde (DE); Rebentisch, Ronald, 10967 Berlin (DE)
(74) Vertreter: Galander, Marcus

(57) **Zusammenfassung**

Die Erfindung betrifft ein elektrisches Kontaktelement (30), insbesondere für ein medizinisches Implantat, umfassend
- eine Federhülse (10) mit einer Durchführung (16) zur Aufnahme eines elektrischen Gegenkontakts (110) in einer Einführrichtung,
- wenigstens eine zumindest bereichsweise quer um die Durchführung (16) angeordnete und zur Durchführung (16) wenigstens bereichsweise offene Nut (18);
- wenigstens ein in der Nut (18) angeordnetes Federelement (52) zur elektrischen Kontaktierung des elektrischen Gegenkontakts (110). Dabei ist wenigstens ein Durchführkanal (20) zur Zuführung des Federelements (52) in die umlaufende Nut (18) vorgesehen, der von einer Außenseite der Federhülse zur wenigstens einen Nut (18) führt

Die Erfindung betrifft weiterhin ein Verfahren zur Herstellung eines Kontaktelements (30) sowie eine Vorrichtung (200) zur Durchführung des Verfahrens.

## Beschreibung

Die Erfindung betrifft ein elektrisches Kontaktelement und ein Verfahren zur Herstellung eines elektrischen Kontaktelements nach den Oberbegriffen der unabhängigen Ansprüche.

Medizinische Implantate, die in den menschlichen oder tierischen Körper einführbar sind und die eine elektrische Versorgungsspannung benötigen, erfordern eine besonders sichere und dauerhafte elektrische Kontaktierung. Solche Implantate sind beispielsweise Herzschrittmacher, Defibrillatoren, Neuro-Stimulatoren und dergleichen.

Aus der DE 690 27 846 T2 ist ein elektrisches Kontaktelement für in den menschlichen oder tierischen Körper einführbare medizinische Implantate bekannt, bei dem eine elektrische Verbindung über eine Torusfeder und einen Pin eines Steckkontakts hergestellt wird. Die Torusfeder ist in eine Nut einer Buchse eingelegt, wobei der elektrische Kontakt zwischen dem Pin und dem Innenumfang der Torusfeder ausgebildet ist.

Die Fertigung von Torusfedern, insbesondere geschlossener Torusfedern, und deren Montage durch Einführen in die umlaufende Nut entsprechender Federhülsen ist aufwendig, schwer automatisierbar und daraus resultierend entsprechend kostenintensiv.

Der Erfindung liegt die Aufgabe zugrunde, ein elektrisches Kontaktelement zu schaffen, das zumindest weitgehend automatisiert herstellbar ist.

Weitere Aufgaben der Erfindung sind, ein automatisiertes Verfahren zur Herstellung eines solchen Kontaktelements sowie eine Vorrichtung zur Durchführung des Verfahrens anzugeben.

Die Aufgaben werden erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Nach einem ersten Aspekt der Erfindung wird ein elektrisches Kontaktelement, insbesondere für ein medizinisches Implantat zum Einführen in den menschlichen oder tierischen Körper, vorgeschlagen, umfassend:
- wenigstens eine Federhülse mit einer Durchführung zur Aufnahme eines elektrischen Gegenkontakts in einer Einführrichtung;
- wenigstens eine um die Durchführung zumindest quer um die Durchführung angeordnete und zur Durchführung wenigstens bereichsweise offene Nut;
- wenigstens ein in der Nut angeordnetes Federelement zur elektrischen Kontaktierung des elektrischen Gegenkontakts,
   wobei wenigstens ein Durchführkanal zur Zuführung des Federelements in die wenigstens eine Nut vorgesehen ist, der von einer Außenseite der Federhülse zur wenigstens einen Nut führt.

Das eingeführte Federelement kann zumindest an den zur Durchführung offenen Bereichen als elektrische Kontaktfläche für einen in die Durchführung eingesteckten Gegenkontakt bilden, die mit Federkraft an dem Gegenkontakt anliegt. Zweckmäßigerweise liegt die Nut in einer konstanten axialen Höhe bezogen auf die Längsachse der Durchführung. Denkbar ist jedoch auch, dass die Nut eine Steigung aufweisen kann. Die Nut kann um die Durchführung umlaufend sein oder auch in einzelne Nutabschnitte unterteilt sein. Diese können miteinander verbunden sein oder getrennt sein. Die Nut kann ein oder auch mehrere Durchführkanäle aufweisen. Ebenso können für jeden Nutabschnitt ein oder mehrere Durchführkanäle vorgesehen sein.

Vorteilhaft kann der Durchführkanal tangential zur Nut angeordnet sein. Dadurch kann das Federelement besonders einfach geführt werden, insbesondere, wenn die Nut und die Durchführung herum umlaufend angeordnet ist. Alternativ kann der Durchführkanal radial auf die Nut zulaufen. Ebenso sind Zwischenstufen der Orientierung des Durchführkanals zur Nut zwischen tangentialem und radialem Zulaufen zur Nut denkbar. Ebenso ist denkbar, dass, bei mehreren Durchführkanälen für dieselbe Nut oder zugehörigen Nutabschnitten für einen zusammengehörigen elektrischen Kontaktbereich, die Durchführkanäle gleiche oder unterschiedliche Orientierungen zur Nut bzw. Nutabschnitten aufweisen.

In einer Ausgestaltung kann das freie Ende des Federelements durch eine zweiten radialen Durchführkanal an einer beliebigen Stelle am Umfang der Federhülse herausgeführt sein, um einen nur teilweise umlaufenden Federkontakt herzustellen. Im Falle der Verwendung nur eines Federelements kann dieses auch ein nicht komplett geschlossenes Torusfederelement sein, das beispielsweise durch einen ersten Durchführkanal eingeführt und in einem zusätzlichen weiteren, tangential zur Nut verlaufenden Durchführkanal wieder aus der Nut herausgeführt werden kann. Dieser zweite Durchführkanal kann beispielsweise ungefähr parallel zum ersten Durchführkanal geführt sein. Weitere Ausgestaltungen sehen zwei oder mehrere Federelemente vor, so dass diese im Wesentlichen langgestreckt vorliegen, aber auch eine leichte Krümmung aufweisen können. Die Kanäle zu Einführen und Herausführen des Federelements können dabei weitgehend fluchtend bzw. leicht verwinkelt angeordnet sein. Vorstellbar sind auch beispielsweise drei langgestreckte Federelemente, welche jeweils 120° zwischen benachbarten Federelementen einschließen.

Vorteilhaft kann das Federelement automatisch zugeführt werden und gegebenenfalls vorher verschränkt werden. Ein manuelles Einlegen einer fertig konfektionierten Torusfeder in die umlaufende Nut ist nicht notwendig. Das Federelement kann günstigerweise eine offene, nicht kreisförmig geschlossene Drahtwendel sein. Das Federelement kann beispielsweise aus Platin-Iridium gebildet sein.

Eine Kontaktvorrichtung kann z. B. mehrere derartige Kontaktelemente mit Federhülsen in einer axialen Richtung aneinandergereiht aufweisen, wobei die umlaufenden Nuten parallel zueinander angeordnet sind, so dass ein Gegenkontakt mit voneinander elektrisch isolierten Kontaktbereichen mit den jeweiligen Federelementen der jeweiligen Federhülsen in Kontakt kommen kann.

Durch die besondere Gestaltung der Federhülse mit tangential auf die umlaufende Nut zulaufendem Durchführkanal kann eine in einem kontinuierlichen Verfahren gewickelte Drahtwendel vollautomatisch als verschränktes und abgelängtes Federelement zugeführt und mit einem Ende mit der Federhülse fügetechnisch verbunden werden.

In einer günstigen Ausgestaltung kann das Federelement an wenigstens einem Punkt mit der Federhülse fest verbunden sein. Vorteilhaft kann das Federelement an einem Punkt, z. B. im Bereich des Durchführkanals, mit der Federhülse verschweißt sein. Insbesondere kann das Federelement mit der Federhülse laserverschweißt sein, was ein besonders genaues Platzieren des Schweißpunkts erlaubt.

In einer weiteren günstigen Ausgestaltung kann das Federelement so weit in die umlaufende Nut der Federhülse eingeschoben sein, das ein vorlaufendes freies Ende des Federelements an einen nachlaufenden Bereich des Federelements anstößt. Damit kann eine große Kontaktfläche für einen elektrischen Gegenkontakt, z. B. einen Pin, bereitgestellt werden. Das Federelement kann günstigerweise eine offene, nicht kreisförmig geschlossene Drahtwendel sein.

In einer weiteren günstigen Ausgestaltung kann das Federelement aus dem tangentialen Durchführkanal herausstehen. Das herausstehende Ende des Federelements kann zur elektrischen Kontaktierung der Federhülse selbst eingesetzt werden. Alternativ kann das Federelement bündig mit dem Durchführkanal abschließen und die Federhülse an anderer Stelle elektrisch kontaktiert werden.

Besonders bevorzugt kann die Federhülse zweiteilig oder mehrteilig aufgebaut sein, wobei die Nut in der Trennebene liegen kann. In dieser Ausführungsform ist es besonders bevorzugt, dass die Nut im zusammengefügten Zustand der beiden Federhülsenteile einen Hinterschnitt aufweist, so dass im zusammengesetzten Zustand der mehrteiligen Federhülse das Federelement um mehr als die Hälfte ihres Umfangs durch die Nut umschlossen ist.

Vorteilhaft kann das Federelement eine Drahtwendel sein. So kann die Drahtwendel ähnlich hergestellt werden wie Wendeln für Herzschrittmacherelektrodenzuleitungen, d.h. eine solche Wendel kann aus einem Draht oder mehreren einzelnen Drähten gewickelt und in größeren Längeneinheiten vorgefertigt werden. Dies erlaubt eine besonders wirtschaftliche Herstellung des Federelements.

Gemäß einem weiteren Aspekt der Erfindung wird ein Verfahren zur Herstellung eines erfindungsgemäßen Kontaktelements vorgeschlagen, wobei eine Drahtwendel über ein Führungselement in eine Federhülse mit radialem Durchführkanal transportiert wird und als Federelement durch den radialen Durchführkanal in eine Nut eingeschoben und auf eine gewünschte Länge gekürzt wird. Dies erlaubt eine wirtschaftliche Herstellung eines Kontaktelements.

Nach einem günstigen Verfahrensschritt kann die Drahtwendel so weit in die Nut eingeschoben werden, bis ein vorlaufendes freies Ende des Federelements an einen nachlaufenden Bereich des Federelements anstößt. Damit kann eine größtmögliche elektrische Kontaktfläche bereitgestellt werden. Alternativ kann das vorlaufende freie Ende der Drahtwendel durch einen zweiten radialen Ausgangskanal herausgeführt werden, wobei die Position des Ausgangskanals beliebig gewählt werden kann. Dieser Ausgangskanal verläuft in entgegen gesetzter tangentialer Richtung zur umlaufenden Nut. Mit Hilfe dieses radialen Ausgangskanals ist es möglich, ein nur teilweise umlaufendes Federelement herzustellen.

Nach einem günstigen Verfahrensschritt kann die Drahtwendel aus einem Wickelprozess über eine Schränkstufe zum Führungselement transportiert werden.

In diesem Fall kann die Drahtwendel ähnlich hergestellt werden wie Wendeln für Herzschrittmacherelektrodenzuleitungen, d.h. diese Wendel kann aus mehreren einzelnen Drähten gewickelt und kann in größeren Längeneinheiten vorgefertigt werden. Von diesen größeren Längeneinheiten können z. B. mittels eines Laserschneidverfahrens Abschnitte abgetrennt werden, die so lang sind, dass sie - durch den tangentialen Durchführkanal eingeführt - die umlaufende Nut völlig ausfüllen und am anderen Ende noch etwas aus dem Durchführkanal herausragen

Einmal in die Federhülse eingeführt, kann das herausragende Ende der Drahtwendel bzw. des Federelements durch einen Laserschweißpunkt in dieser Position lagefixiert werden.

Nach einem weiteren Aspekt der Erfindung wird eine Vorrichtung zur Durchführung des erfindungsgemäßen Verfahrens vorgeschlagen, mit einem ein Transportmechanismus, um eine Drahtwendel über ein Führungselement zu einer Federhülse zu transportieren und durch einen radialen Durchführkanal in eine Nut in der Federhülse einzuschieben, mit einem Schneidmechanismus, um ein Federelement von der Drahtwendel abzutrennen und auf eine gewünschte Länge zu kürzen, sowie mit einer Verbindungseinheit, um das Federelement an wenigstens einem Punkt mit der Federhülse fest zu verbinden.

Vorteilhaft kann eine Schränkstufe vorgesehen sein, um die Drahtwendel vor dem Einschieben in die Nut zu verschränken.

Vorteilhaft sind Kosteneinsparungen durch einen Herstellungsprozess mit hoher Automatisierbarkeit möglich. Die Drahtwendel kann direkt aus Wickelprozess über die Schränkstufe, z. B. aus zwei rotierenden Rollen, transportiert und über ein Führungselement in die Federhülse mit radialem Durchführkanal laufen.

Das eingeführte Federelement kann als Verdrahtungselement genutzt und an einem Punkt automatisch mit der Federhülse verschweißt und in entsprechender Länge getrennt werden. Alternativ kann das Federelement mit der Federhülse bündig verschweißt und abgelängt werden.

Die Erfindung ist nachfolgend beispielhaft, anhand von in Zeichnungen dargestellten Ausführungsbeispielen, näher erläutert. Es zeigen in schematischer Darstellung:
- Fig. 1: eine schematische Darstellung einer Kontaktvorrichtung mit Kontaktelementen nach einer ersten Ausgestaltung der Erfindung;
- Fig. 2: eine Ansicht einer ersten Ausgestaltung einer Federhülse mit einer seitlichen Bohrung als tangential auf eine Nut in der Federhülse zulaufendem Durchführkanal;
- Fig. 3: einen Schnitt die Federhülse aus Figur 2;
- Fig. 4: zwei getrennte Teilstücke der Federhülse aus Fig. 2;
- Fig. 5: eine Ansicht einer weiteren Ausgestaltung einer Federhülse mit stirnseitlicher Bohrung als tangential auf eine Nut in der Federhülse zulaufendem Durchführkanal, dargestellt in zwei getrennten Teilstücken;
- Fig. 6: einen Schnitt durch die getrennten Teilstücke der Federhülse aus Fig. 5;
- Fig. 7: eine teilaufgeschnittene Federhülse mit in eine Nut eingeführtem Federelement;
- Fig. 8: schematisch eine elektrische Kontaktfläche eines Gegenkontakts in Kontakt mit einer Federhülse;
- Fig. 9: eine günstige Ausgestaltung einer Federhülse mit Federelement;
- Fig. 10: eine günstige Ausgestaltung einer Federhülse mit zwei Federelementen;
- Fig. 11: eine günstige Ausgestaltung einer Federhülse mit drei Federelementen; und
- Fig. 12: schematisch eine vorteilhafte Ausgestaltung einer Vorrichtung zur Herstellung eines Kontaktelements.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

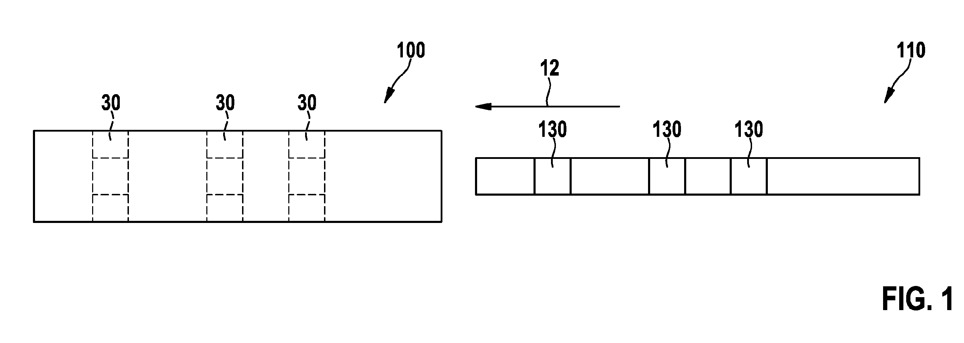

Figur 1 zeigt eine schematische Ansicht einer Ausgestaltung einer Kontaktvorrichtung 100 mit einer Mehrzahl, beispielsweise drei, von elektrischen Kontaktelementen 30. Details eines medizinischen Implantats, mit dem die Kontaktvorrichtung 100 und ein dieser zugeordneter elektrischer Gegenkontakt 110 gekoppelt sind, wie z. B. angeschlossene Komponenten, elektrische Bauteile und dergleichen, sind nicht dargestellt.

Der elektrische Gegenkontakt 110, beispielsweise ein Pin, ist dazu vorgesehen, in Richtung 12 einer Längsachse in die Kontaktvorrichtung 100 eingesteckt zu werden. Dabei wird der elektrische Gegenkontakt 110 durch die Kontaktelemente 30 durchgeführt. Der elektrische Gegenkontakt 110 weist an seiner Außenseite eine Mehrzahl, beispielsweise drei, von Kontaktflächen 130 auf, die mit den Kontaktelementen 30 in Kontakt kommen, wenn der elektrische Gegenkontakt 110 in die Kontaktvorrichtung 100 eingeschoben ist.

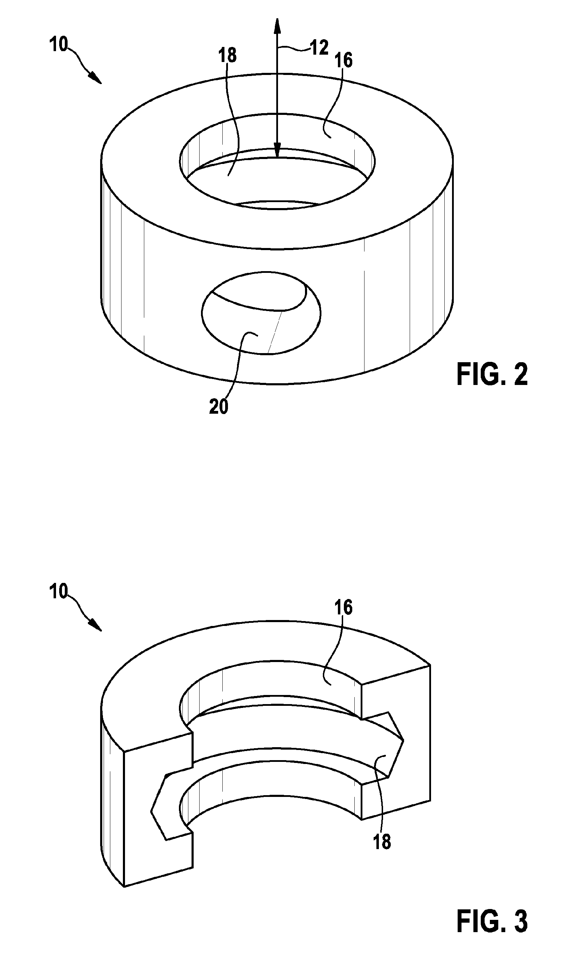

Die Figuren 2, 3 und 4 zeigen zur Erläuterung der Erfindung verschiedene Ansichten und Schnitte einer erste Ausgestaltung eines elektrischen Kontaktelements 30, insbesondere für ein medizinisches Implantat, umfassend eine Federhülse 10 mit einer Durchführung 16 zur Aufnahme eines elektrischen Gegenkontakts (nicht dargestellt) in einer Einführrichtung 12.

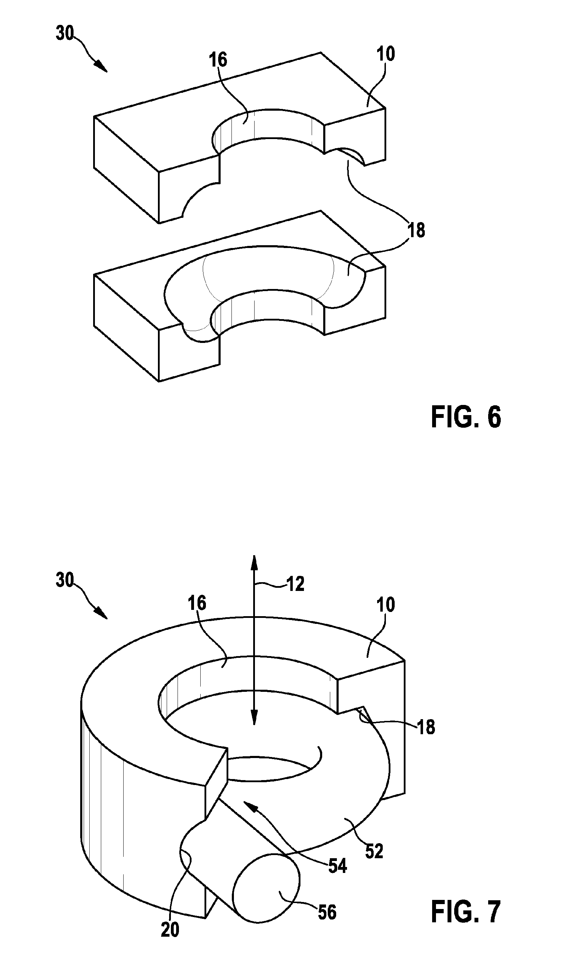

Um die sich in axialer Richtung 12 erstreckende Durchführung 16 ist eine um die Durchführung 16 umlaufende und zur Durchführung 16 offene Nut 18 angeordnet, welche in einer Ebene quer zur Richtung 12 liegt. Die Nut 18 ist dazu vorgesehen, ein Federelement 52 (Fig. 7) aufzunehmen, um den elektrischen Gegenkontakt (nicht dargestellt) elektrisch zu kontaktieren.

Tangential zur umlaufenden Nut 18 in der Federhülse 10 ist eine Bohrung als Durchführkanal 20 zur Zuführung des Federelements 52 in die umlaufende Nut 18 angeordnet. Der Durchführkanal 20 läuft von der Außenseite der Federhülse 10 auf die Nut 18 zu und ist in derselben Ebene wie die Nut 18 angeordnet. Die Nut 18 kann z. B. ein prismatisches (eckiges) Profil aufweisen.

In der hier dargestellten Ausführung ist die Federhülse 10 zweiteilig aufgebaut. Die Trennung der Federhülse erfolgt in der Ebene quer zur Richtung 12 und verläuft genau auf der Mittellinie der umlaufenden Nut 18, die durch Zusammensetzen der beiden Federhülsenteile entsteht. Mit anderen Worten liegt die Nut 18 in der Trennebene, in der die Federhülse 10 getrennt ist, und wird durch das Zusammensetzen komplettiert. Konkret sind beide Teile der Federhülse 10 mit je einem sich ergänzenden Abschnitt der Nut 18 versehen. Das Besondere an der hier dargestellten zweigeteilten Federhülse 10 ist, dass im zusammengefügten Zustand der beiden Federhülsenteile eine Nut 18 für das Federelement 52 durch zwei Hinterschnitte entsteht. Im zusammengesetzten Zustand ist das Federelement 52 also um mehr als die Hälfte seines Umfangs durch die Nut 18 umschlossen Dadurch wird verhindert, dass das Federelement 52 bei der Betätigung des Federkontakts 10 aus der Nut rutschen kann, was bei einem offenen Federelement 52 von herausragender Bedeutung ist. Herstellbar sind diese zweiteiligen Federhülsen 10 beispielsweise kostengünstig und damit besonders wirtschaftlich mittels urformender Verfahren wie Spritzen oder Gießen.

Die Federhülse 10 ist z. B. zylinderförmig ausgebildet. Das Kontaktelement kann auch mehrere derartige Federhülsen 10 aufweisen, die konzentrisch zur Symmetrieachse und in Richtung 12 aufeinanderfolgend angeordnet sind (nicht dargestellt).

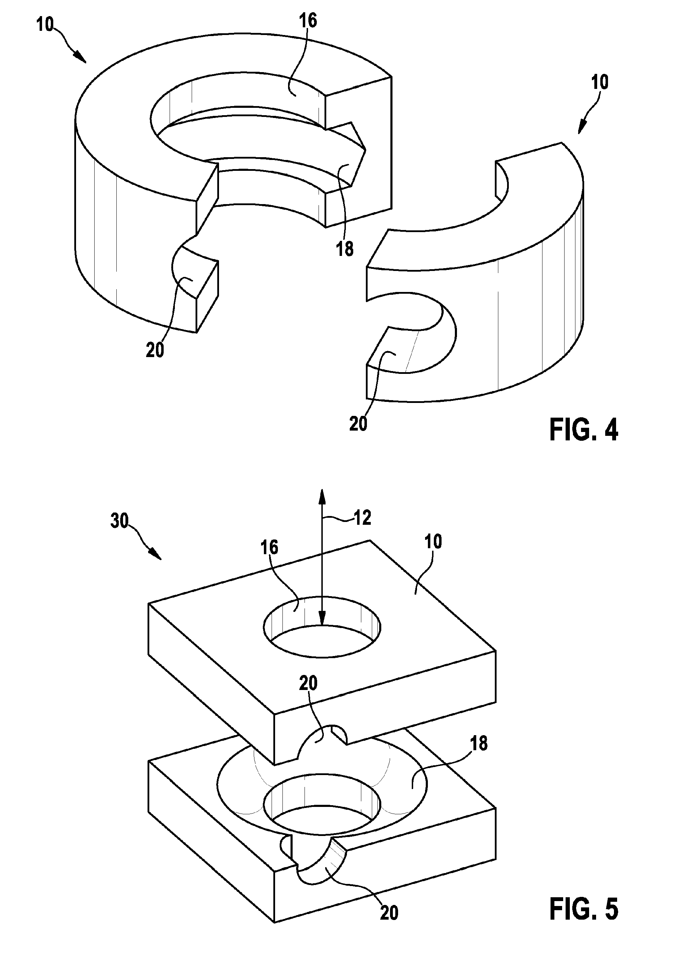

Die Figuren 5 und 6 zeigen eine weitere Ausgestaltung eines Kontaktelements 30, bei dem die Federhülse 10 einen rechteckigen, insbesondere quadratischen, Querschnitt hat. Die Nut 18 kann z. B. ein gerundetes Profil aufweisen. Der Durchführkanal 20 ist als Bohrung in einer Stirnfläche der Federhülse 10 ausgeführt, welche tangential auf die Nut 18 im Inneren der Federhülse 10 zuläuft.

Figur 7 zeigt eine teilweise aufgeschnittene Federhülse 10 mit in eine Nut 18 eingeführtem Federelement 52. Die Nut 18 ist um eine Durchführung 16 konzentrisch umlaufend angeordnet. Wird ein elektrischer Gegenkontakt, z. B. ein Pin (nicht dargestellt), in Richtung 12 in die Durchführung 16 eingeführt, kommt dieser mit dem Federelement 52 in Berührung und stellt eine elektrische Verbindung zwischen Federelement 52 und Gegenkontakt her. Das Federelement 52 ist zur Fixierung seiner Lage an wenigstens einem Punkt mit der Federhülse 10 fest verbunden, z. B. verschweißt.

Das Federelement 52 ist so weit in die umlaufende Nut 18 der Federhülse 10 eingeschoben ist, dass ein vorlaufendes freies Ende 54 des Federelements 52 an einen nachlaufenden Bereich des Federelements 52 anstößt.

In der gezeigten Ausgestaltung steht das Federelement 52 aus dem tangentialen Durchführkanal 20 heraus und kann Anschluss für eine elektrische Kontaktierung der Federhülse 10 verwendet werden. Alternativ kann das Federelement 52 bündig mit dem Durchführkanal 20 abschließen (nicht dargestellt).

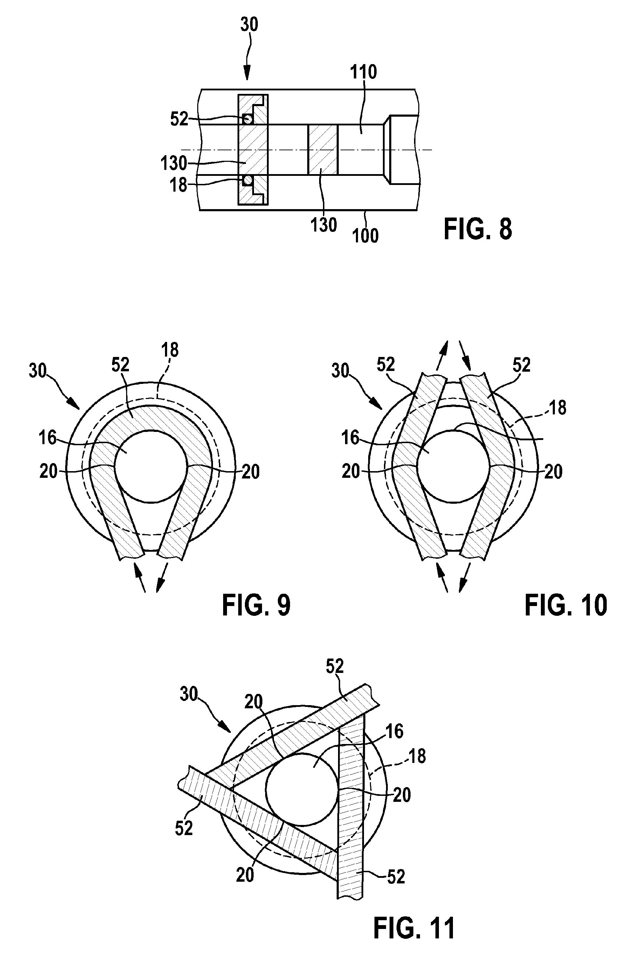

Fig. 8 zeigt schematisch eine elektrische Kontaktfläche 130 eines Gegenkontakts 110 in Kontakt mit einer quer geteilten Federhülse 30. Die Federhülse 30 kann vorteilhaft aus scheibenartigen Teilelementen zusammengesetzt sein, wobei im zusammengesetzten Zustand eine Nut 18 für das Federelement 52 gebildet wird.

Die Figuren 9, 10 und 11 illustrieren beispielhaft günstige Ausgestaltungen einer Federhülse 30 mit mehreren Kanälen 20, die zum Durchführen eines Federelements 30 dienen. Figur 9 zeigt dabei zwei nebeneinander liegende Durchführkanäle 20. Durch einen der Durchführkanäle 20 wird ein Federelement 52 eingeschoben, das durch den benachbarten Durchführkanal 20 mit seinem freien Ende austreten kann. Das Federelement 52 kann entsprechend abgelängt werden und gegebenenfalls an der Federhülse 30 fixiert werden, wie vorstehend bereits beschrieben wurde. Das Federelement 52 umschließt die Durchführung 16 dabei weitgehend.

Figur 10 zeigt eine günstige Ausgestaltung einer Federhülse 30 mit zwei Federelementen 52, wobei jede auf der einen Seite der Federhülse 30 durch einen Einführkanal 20 in die Federhülse 30 eingeschoben ist und an der gegenüberliegenden Seite der Federhülse 30 durch einen Durchführkanal 20 mit ihrem freien Ende mehr oder weniger weit aus der Federhülse 30 herausragen kann. Die Federelemente 52 können entsprechend abgelängt werden und gegebenenfalls an der Federhülse 30 fixiert werden, wie vorstehend bereits beschrieben wurde. Die beiden Federelemente 52 umschließen die Durchführung 16 bzw. einen elektrischen Gegenkontakt nicht, sondern berühren eine elektrische Kontaktfläche eines Gegenkontakts an zwei diametral gegenüberliegenden Seiten.

Figur 11 zeigt eine günstige Ausgestaltung einer Federhülse 30 mit drei um 120° um die Durchführung 16 versetzt angeordneten Federelementen 52, die tangential und im wesentlichen in gestreckter Form durch separate Durchführkanäle 20 in die Federhülse 30 eingeführt sind.

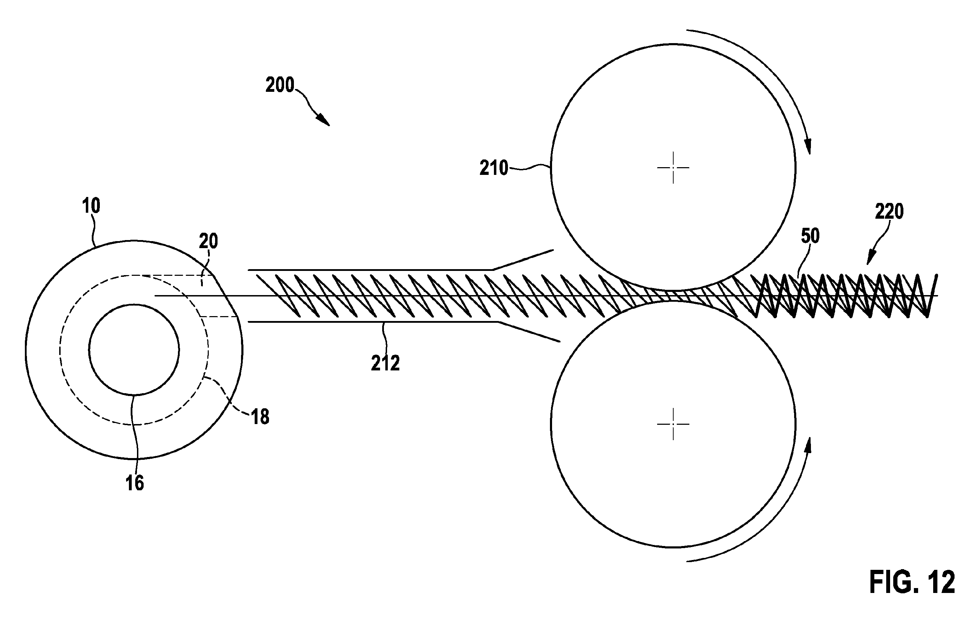

Figur 12 illustriert schematisch eine Vorrichtung 200 zur Herstellung eines Kontaktelements 30.

Die Vorrichtung 200 umfasst einen (nur schematisch angedeuteten) Transportmechanismus 220, um eine Drahtwendel 50 über ein Führungselement 212 zu einer Federhülse 10, die in einem Arbeitsbereich der Vorrichtung 200 bereitgehalten wird, zu transportieren und durch einen radialen Durchführkanal 20 in der Wand der Federhülse 10 in eine Nut 18 in der Federhülse 10 einzuschieben, einen Schneidmechanismus (nicht dargestellt), um ein Federelement 52 (Figur 7) von der Drahtwendel 50 abzutrennen und auf eine gewünschte Länge zu kürzen, sowie eine Verbindungseinheit, um das Federelement 52 (Figur 7) an wenigstens einem Punkt mit der Federhülse 10 fest zu verbinden, z. B. zu verschweißen. Vorteilhaft kann das Schneiden der Drahtwendel 50 und das Befestigen des Federelements 52 mit einem Laser erfolgen, so dass der Schneidmechanismus und die Verbindungseinheit auch identisch sein können.

Der tangentiale Durchführkanal 20 liegt in derselben Ebene wir die Nut, so dass beim Transport der Drahtwendel 50 diese mit ihrem vorlaufenden Ende durch den tangentialen Durchführkanal 20 in der Wand der Federhülse 10 in die Nut 18 innerhalb der Federhülse 10 mit einer gewünschten Länge eingeschoben werden kann, maximal bis das vorlaufende Ende in der Nut 18 an einen nachlaufenden Bereich der Drahtwendel 50 stößt.

Dann kann das eingeschobene Stück der Drahtwendel 50 auf eine gewünschte Länge gekürzt werden, so dass in der Federhülse 10 ein Federelement 52 (Figur 7) zurückbleibt. Vor oder nach dem Ablängen der Drahtwendel 50 kann das Federelement 52 mit der Federhülse 10 verbunden werden, insbesondere an einem Punkt laserverschweißt werden.

Die Drahtwendel 50 ist dann zum Beschicken der nächsten bereitgestellten Federhülse 10 mit einem Federelement 52 (Figur 7) bereit. Aus der Drahtwendel 50 können eine Vielzahl von Federelementen 52 (Figur 7) gebildet werden, die zum Einbringen in vorbereitete Federhülsen 10 vorgesehen sind, so dass ein kontinuierlicher automatischer Herstellprozess vom Wickeln der Drahtwendel 50 bis und mit zum Einbringen der Federelemente 52 in die Federhülsen 10 möglich ist.

Die Drahtwendel 50 kann direkt aus einem Wickelprozess über eine Schränkstufe 110, beispielsweise zwei gegenläufig drehende Walzen, zwischen denen die Drahtwendel 50 durchgeführt wird, verschränkt und mit einer schematisch angedeuteten Transporteinrichtung 220 zum Führungselement 212 transportiert werden.

Die erfindungsgemäße Vorrichtung 200 erlaubt ein automatisiertes, wirtschaftliches Verfahren zur Herstellung eines Kontaktelements 30 mit Federelement.

## Patentansprüche

1. Elektrisches Kontaktelement (30), insbesondere für ein medizinisches Implantat, umfassend
- eine Federhülse (10) mit einer Durchführung (16) zur Aufnahme eines elektrischen Gegenkontakts (110) in einer Einführrichtung,
- wenigstens eine zumindest bereichsweise quer um die Durchführung (16) angeordnete und zur Durchführung (16) wenigstens bereichsweise offene Nut (18);
- wenigstens ein in der Nut (18) angeordnetes Federelement (52) zur elektrischen Kontaktierung des elektrischen Gegenkontakts (110),
**gekennzeichnet durch**
wenigstens einen Durchführkanal (20) zur Zuführung des Federelements (52) in die wenigstens eine Nut (18), der von einer Außenseite der Federhülse zur wenigstens einen Nut (18) führt

2. Kontaktelement nach Anspruch 1, **dadurch gekennzeichnet, dass** der wenigstens eine Durchführkanal (20) sich quer zur wenigstens einen Nut (16) erstreckt.

3. Kontaktelement nach Anspruch 1 oder 2, **dadurch gekennzeichnet, dass** der wenigstens eine Durchführkanal (20) sich tangential zur wenigstens einen Nut (16) erstreckt.

4. Kontaktelement nach Anspruch 1, **dadurch gekennzeichnet, dass** das Federelement (52) an wenigstens einem Punkt mit der Federhülse (10) fest verbunden ist.

5. Kontaktelement nach einem der vorhergehenden Ansprüche, **dadurch gekennzeichnet, dass** das Federelement (52) so weit in die umlaufende Nut (18) der Federhülse (10) eingeschoben ist, dass ein vorlaufendes freies Ende (54) des Federelements (52) an einen nachlaufenden Bereich des Federelements (52) anstößt.

6. Kontaktelement nach einem der vorhergehenden Ansprüche, **dadurch gekennzeichnet, dass** das Federelement (52) aus dem tangentialen Durchführkanal (20) heraussteht.

7. Kontaktelement nach einem der Ansprüche 1 bis 5, **dadurch gekennzeichnet, dass** das Federelement (52) bündig mit dem Durchführkanal (20) abschließt.

8. Kontaktelement nach einem der Ansprüche 1 bis 5, **dadurch gekennzeichnet, dass** Federhülse (10) zweiteilig oder mehrteilig ist und die Nut (18) in der Trennebene liegt.

9. Kontaktelement nach Anspruch 8, **dadurch gekennzeichnet, dass** die Nut (18) im zusammengefügten Zustand der beiden Federhülsenteile einen Hinterschnitt aufweist, so dass im zusammengesetzten Zustand der mehrteiligen Federhülse (10) das Federelement (52) um mehr als die Hälfte ihres Umfangs durch die Nut (18) umschlossen ist.

10. Kontaktelement nach einem der vorhergehenden Ansprüche, **dadurch gekennzeichnet, dass** das Federelement (52) eine Drahtwendel ist.

11. Federhülse für ein elektrisches Kontaktelement (30) nach einem der vorhergehenden Ansprüche, insbesondere für ein medizinisches Implantat, umfassend
- eine Durchführung (16) zur Aufnahme eines elektrischen Gegenkontakts (110) in einer Einführrichtung,
- wenigstens eine zumindest bereichsweise quer um die Durchführung (16) angeordnete und zur Durchführung (16) wenigstens bereichsweise offene Nut (18);
- wenigstens ein in der Nut (18) angeordnetes Federelement (52) zur elektrischen Kontaktierung des elektrischen Gegenkontakts (110),
**gekennzeichnet durch**
wenigstens einen Durchführkanal (20) zur Zuführung des Federelements (52) in die Nut (18)., der von einer Außenseite der Federhülse zur wenigstens einen Nut (18) führt

12. Verfahren zur Herstellung eines Kontaktelements (30) nach einem der vorhergehenden Ansprüche, wobei eine Drahtwendel (50) über ein Führungselement (212) in einen Durchführkanal (20) einer Federhülse (10) transportiert wird und durch den Durchführkanal (20) in eine Nut (18) eingeschoben und auf eine gewünschte Länge gekürzt wird.

13. Verfahren nach Anspruch 12, wobei die Drahtwendel (50) so weit in die Nut (18) eingeschoben wird, bis ein vorlaufendes freies Ende (54) des Federelements (52) an einen nachlaufenden Bereich des Federelements (52) anstößt.

14. Verfahren nach Anspruch 12 oder 13, wobei die Drahtwendel (50) aus einem Wickelprozess über eine Schränkstufe (210) zum Führungselement (112) transportiert und in der Schränkstufe (210) verschränkt wird.

15. Verfahren nach einem der Ansprüche 12 bis 14, wobei das Federelement (52) wenigstens an einem Punkt mit der Federhülse (10) fest verbunden wird.

16. Vorrichtung (200)zur Durchführung des Verfahrens nach einem der Ansprüche 12 bis 15,
- mit einem ein Transportmechanismus (220), um eine Drahtwendel (50) über ein Führungselement (212) zu einer Federhülse (10) zu transportieren und durch einen radialen Durchführkanal (20) in eine Nut (18) in der Federhülse (10) einzuschieben,
- mit einem Schneidmechanismus, um ein Federelement (52) von der Drahtwendel (50) abzutrennen und auf eine gewünschte Länge zu kürzen, sowie
- mit einer Verbindungseinheit, um das Federelement (52) an wenigstens einem Punkt mit der Federhülse (10) fest zu verbinden.

17. Vorrichtung nach Anspruch 16, wobei eine Schränkstufe (210) vorgesehen ist, um die Drahtwendel (50) vor dem Einschieben in die Nut (18) zu verschränken.
